# EUROPEAN PATENT APPLICATION

(11) **EP 1 557 420 A1**
(43) Date of publication of application: **27.07.2005**
(21) Application number: 03758784.7
(22) Date of filing: 22.10.2003
(51) Int. Cl.: C07D 495/10

(54) **PROCESS FOR PRODUCING OPTICALLY ACTIVE SULFOXIDE**

(30) Priority: 24.10.2002 JP 2002309653
(71) Applicant: Sankyo Company, Limited, Tokyo 103-8426 (JP)
(72) Inventor: Tomori, Hiroshi, c/o Sankyo Company, Ltd, Hiratsuka-shi, Kanagawa 254-0014 (JP); Okachi, Takahiro, c/o Sankyo Company, Ltd, Hiratsuka-shi, Kanagawa 254-0014 (JP); Kobayashi, Keijiro, c/o Sankyo Company, Ltd, Hiratsuka-shi, Kanagawa 254-0014 (JP)
(74) Representative: Gibson, Christian John Robert
(86) International application number: PCT/JP2003/013495
(87) International publication number: WO 2004/037828

(57) **Abstract**

[Object]

The present invention provides a process for preparing optically active cyclic sulfoxides.

[Means for solution]

A process for preparing an optically active cyclic sulfoxide, which is **characterized by** reacting a cyclic thioether with cumene hydroperoxide or isopropylcumyl hydroperoxide in the presence of alcohol, water or a mixture of water and alcohol and in the presence of a complex of an optically active tartaric acid diester and a titanium (IV) alkoxide in an inert solvent.

## Description

### [TECHNICAL FIELD]

The present invention relates to a process for preparing an optically active cyclic sulfoxide that serves as a synthesis intermediate of a superior neurokinin receptor antagonist (EP0987269).

### [BACKGROUND ART]

Examples of known methods for synthesizing cyclic sulfoxides having an excess of enantiomer capable of serving as important synthesis intermediates of neurokinin receptor antagonists include a method in which asymmetric oxidation is carried out directly using an asymmetric oxidation agent in accordance with the method of F.A. Davis et al., and a method in which a racemic sulfoxide obtained by ordinary oxidation is optically resolved by the diastereomer method (US Patent No. 6159967 (Columns 254-257, Preparation 6) and T. Nishi, et al., Tetrahedron Asymmetry, 1998, 9, 2567-2570).

In the former method, namely the method that uses an asymmetric oxidation agent, although the yield is high (95%) and cyclic sulfoxide is obtained with a high enantiomeric excess (96%), this method is not suitable as an industrial production process due to the fact that it is an equimolar reaction, and due to the asymmetric oxidation agent being expensive, and difficult to recover.

On the other hand, in the latter method, namely the optical resolution by the diastereomer method, although cyclic sulfoxide with a high enantiomeric excess (99% or more) is obtained, this method is also not suitable as an industrial production process from the economical viewpoint due to the low yield (30-36%) as a result of having to discard half of the sulfoxide, namely the reverse side enantiomer.

Moreover, a method of synthesizing cyclic sulfoxides is known that uses tert-butylhydroperoxide as an oxidation agent by combining an optically active diol, such as diethyl tartrate or binaphthol, titanium (IV) isopropoxide and water (T. Nishi et al., Tetrahedron Asymmetry, 1998, 9, 2567-2570). However, the yields of cyclic sulfoxide with this method are 20% and 46%, respectively, and the enantiomeric excesses are 54% and 17%, respectively, with both being extremely low, thereby making this method unsuitable as an industrial production process.

### [DISCLOSURE OF THE INVENTION]

In order to solve these problems, the inventors of the present invention conducted extensive research on an industrial process for producing cyclic sulfoxides. As a result, it was found that by carrying out an oxidation reaction using a specific oxidation agent (cumene hydroxyperoxide or isopropyl cumyl hydroperoxide) in the presence of alcohol, water or a mixture of alcohol and water and using a complex of an optically active tartaric acid diester and a titanium (IV) alkoxide as an asymmetric catalyst, a cyclic sulfoxide can be obtained at high yield (90-95%) and with a high enantiomeric excess (87-89% or higher), thereby leading to completion of the present invention.

The present invention relates to (1) a process for preparing a compound of formula (1): 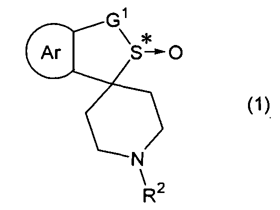 [wherein G¹ represents a C₁-C₆ alkylene group; Ar represents a C₆-C₁₀ aryl group which may be substituted by one or more group(s) selected from Substituent group α or a 5 to 7-membered heteroaryl group containing 1 to 3 sulfur atoms, oxygen atoms and/or nitrogen atoms which may be substituted by one or more group(s) selected from Substituent group α; R² represents a hydrogen atom or an amino protecting group; and Substituent group α consists of a C₁-C₆ alkyl group, a C₁-C₆ alkoxy group and a halogen atom; and * represents an asymmetrical center] or an acid addition salt thereof, which is characterized by reacting a compound of formula (2): 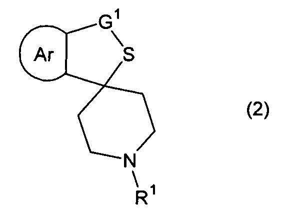 [wherein G¹ and Ar have the same meanings as defined above; R¹ represents an amino protecting group] with cumene hydroperoxide or isopropylcumyl hydroperoxide in the presence of alcohol, water or a mixture of water and alcohol and in the presence of a complex of an optically active tartaric acid diester and a titanium (IV) alkoxide in an inert solvent.

Of the above processes, preferred are:
(2) the process comprising a step of removing R¹, if desired, and carrying out optical resolution, after the oxidation step;
(3) the process comprising a step of removing R¹, followed by carrying out optical resolution by the diastereomer method, after the oxidation step;
(4) the process wherein G¹ is a C₁-C₃ straight alkylene group;
(5) the process wherein G¹ is a methylene group;
(6) the process wherein Ar is a phenyl group which may be substituted by one or more group(s) selected from Substituent group α;
(7) the process wherein Ar is a phenyl group or a phenyl group substituted by 1 or 2 groups selected from the group consisting of fluorine atoms, chlorine atoms, methyl, ethyl, methoxy and ethoxy groups;
(8) the process wherein Ar is a phenyl group;
(9) the process wherein R¹ is a C₁-C₄ alkanoyl, trifluoroacetyl, methoxyacetyl, benzoyl, 1-naphthoyl, 2-naphthoyl, anisoyl, nitrobenzoyl, C₁-C₄ alkoxycarbonyl, 2,2,2-trichloroethoxycarbonyl, triethylsilylmethoxycarbonyl, 2-(trimethylsilyl)ethoxycarbonyl, vinyloxycarbonyl, allyloxycarbonyl, benzyloxycarbonyl or nitrobenzyloxycarbonyl group;
(10) the process wherein R¹ is trifluoroacetyl, methoxycarbonyl, ethoxycarbonyl or tert-butoxycarbonyl;
(11) the process wherein R² is a hydrogen atom;
(12) the process wherein the titanium (IV) alkoxide is titanium (IV) methoxide, titanium (IV) ethoxide, titanium (IV) propoxide or titanium (IV) isopropoxide;
(13) the process wherein the titanium (IV) alkoxide is titanium (IV) isopropoxide;
(14) the process wherein the optically active tartaric acid diester is dimethyl (+)- or (-)-tartrate, diethyl (+)- or (-)-tartrate, diisopropyl (+)- or (-)-tartrate, dibutyl (+)- or (-)-tartrate or di-tert-butyl (+)- or (-)-tartrate;
(15) the process wherein the optically active tartaric acid diester is diethyl (+)- or (-)-tartrate or diisopropyl (+)- or (-)-tartrate;
(16) the process wherein the optically active tartaric acid diester is diisopropyl (+)- or (-)-tartrate;
(17) the process wherein the optical resolution agent used in the optical resolution is an optically active sulfonic acid or optically active carboxylic acid;
(18) the process wherein the optical resolution agent used in the optical resolution is (+)- or (-)-camphor-10-sulfonic acid, (+)- or (-)-tartaric acid, diacetyl (+)- or (-)-tartaric acid, dibenzoyl (+)- or (-)-tartaric acid, (+)- or (-)-mandelic acid or (+)- or (-)-malic acid;
(19) the process wherein the compound of the formula (1) is a compound having the S configuration and the optically active tartaric acid diester is dimethyl (-)-tartrate, diethyl (-)-tartrate, diisopropyl (-)-tartrate, dibutyl (-)-tartrate or di-tert-butyl (-)-tartrate and the optical resolution agent used in the case where optical resolution is carried out is (-)-camphor-10-sulfonic acid, (+)-tartaric acid, dibenzoyl (+)-tartaric acid or (+)-mandelic acid;
(20) the process wherein the optically active tartaric acid diester is diethyl (-)-tartrate or diisopropyl (-)-tartrate; and
(21) the process wherein the optically active tartaric acid diester is diisopropyl (-)-tartrate.

Further, the present invention is directed to a process for preparing a compound of the following formula (4): 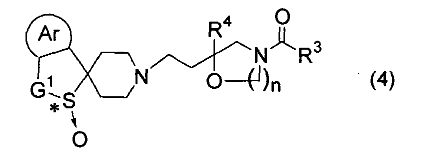 (wherein G¹ represents a C₁-C₆ alkylene group; Ar represents a C₆-C₁₀ aryl group which may be substituted by one or more group(s) selected from Substituent group α or a 5 to 7-membered heteroaryl group containing 1 to 3 sulfur atoms, oxygen atoms and/or nitrogen atoms which may be substituted by one or more group(s) selected from Substituent group α; Substituent group α consists of C₁-C₆ alkyl groups, C₁-C₆ alkoxy groups and halogen atoms; R³ represents a phenyl group substituted by from 1 to 3 groups selected from hydroxyl groups, C₁-C₄ alkoxy groups, halogenated C₁-C₄ alkyl groups and a tetrazolyl group; R⁴ represents a phenyl group substituted by 1 or 2 halogen atoms; n represents 1 or 2; and * represents an asymmetrical center) or a pharmacologically acceptable salt thereof, which substantially consists of the following step A and step B: {wherein step A is a step to prepare the compound of formula (1) : 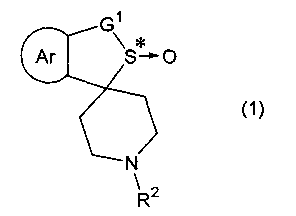 [wherein G¹, Ar and * have the same meanings as defined above; and R² represents a hydrogen atom or a group which is the same group as the group as defined for R¹] or an acid addition salt thereof, by reacting a compound of formula (2): 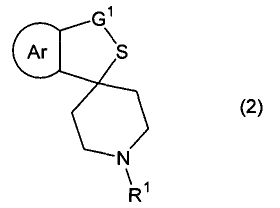 [wherein G¹ and Ar have the same meanings as defined above; and R¹ represents an amino protecting group] with cumene hydroperoxide or isopropylcumyl hydroperoxide in the presence of alcohol, water or a mixture of water and alcohol and in the presence of a complex of an optically active tartaric acid diester and a titanium (IV) alkoxide in an inert solvent; and step B is a step to prepare the compound of formula (4) by removing R² in the case where R² of the compound (1) obtained in step A is an amino protecting group and reacting the compound (1), wherein R² is a hydrogen atom, with the compound of formula (3): 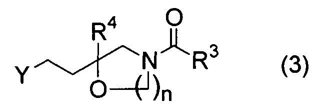 (wherein R³, R⁴ and n have the same meanings as defined above; and Y represents a leaving group).}

Of the above processes, preferable processes are:
the process in which G¹ is a methylene group;
the process in which Ar is a phenyl group;
the process in which R¹ is trifluoroacetyl, methoxycarbonyl, ethoxycarbonyl, tert-butoxycarbonyl or benzyloxycarbonyl;
the process in which R² is a hydrogen atom;
the process in which Y is a halogen atom, a lower alkanesulfonyloxy group, a halogeno lower alkanesulfonyloxy group or an arylsulfonyloxy group;
the process in which n is 2;
the process in which R³ is 3,5-bis(trifluoromethyl)phenyl, 3,4,5-trimethoxyphenyl, 3-hydroxy-4,5-dimethoxyphenyl, 4-hydroxy-3,5-dimethoxyphenyl or 2-methoxy-5-(1-tetrazolyl)phenyl; and
the process in which R⁴ is a phenyl group substituted by 1 or 2 fluorine atoms or chlorine atoms.

In the above general formulae (1), (2), (3) and (4), the "C₁-C₆ alkylene group" in the definition of G¹ can be, for example, a straight or branched alkylene group such as a methylene, ethylene, trimethylene, propylene, tetramethylene, 1-methyltrimethylene, 2-methyltrimethylene, 1,1-dimethylethylene, pentamethylene, 1,1-dimethyltrimethylene, 2,2-dimethyltrimethylene, 1,2-dimethyltrimethylene or hexamethylene group, preferably a C₁-C₃ straight or branched alkylene group, more preferably a C₁-C₃ straight alkylene group, still more preferably a methylene or ethylene group, most preferably a methylene group.

The aryl moiety of the "C₆-C₁₀ aryl group which may be substituted by one or more group(s) selected from Substituent group α" in the definition of Ar can be, for example, a phenyl or naphthyl group, preferably a phenyl group.

Further, the above "C₆-C₁₀ aryl group" may be condensed with a C₃-C₁₀ cycloalkyl (preferably C₅-C₆ cycloalkyl) group.

In the case where Ar represents "a C₆-C₁₀ aryl group substituted by one or more group(s) selected from Substituent group α", it is preferably a C₆-C₁₀ aryl group substituted by from 1 to 4 groups selected from Substituent group α, more preferably a C₆-C₁₀ aryl group substituted by from 1 to 3 groups selected from Substituent group α, still more preferably a C₆-C₁₀ aryl group substituted by from 1 to 3 groups selected from the group consisting of fluorine atoms, chlorine atoms, methyl, ethyl, methoxy and ethoxy groups.

The "5- to 7-membered heteroaryl group containing from 1 to 3 sulfur atoms, oxygen atoms and/or nitrogen atoms" moiety of the "5- to 7-membered heteroaryl group containing from 1 to 3 sulfur atoms, oxygen atoms and/or nitrogen atoms which may be substituted by one or more group(s) selected from Substituent group α" in the definition of Ar can be, for example, a furyl, thienyl, pyrrolyl, pyrazolyl, imidazolyl, oxazolyl, isoxazolyl, thiazolyl, isothiazolyl, triazolyl, thiadiazolyl, pyridyl, pyridazinyl, pyrimidinyl, pyrazinyl or azepinyl group, preferably a 5- or 6-membered heteroaryl group containing 1 or 2 sulfur atoms, oxygen atoms and/or nitrogen atoms such as a furyl, thienyl, pyrrolyl, pyrazolyl, imidazolyl, oxazolyl, isoxazolyl, thiazolyl, isothiazolyl, pyridyl, pyridazinyl, pyrimidinyl or pyrazinyl group, more preferably a pyridyl or pyrimidinyl group.

Furthermore, the aforementioned "5- to 7-membered heteroaryl group containing from 1 to 3 sulfur atoms, oxygen atoms and/or nitrogen atoms" may be condensed with another cyclic group [for example, a C₆-C₁₀ aryl (preferably phenyl) or C₃-C₁₀ cycloalkyl (preferably C₅-C₆ cycloalkyl) group and such a group can be, for example, an indolyl, benzofuranyl, benzothienyl, quinolyl, isoquinolyl, quinazolinyl, tetrahydroquinolyl or tetrahydroisoquinolyl group.

In the case where Ar represents a "5- to 7-membered heteroaryl group containing from 1 to 3 sulfur atoms, oxygen atoms and/or nitrogen atoms substituted by one or more group(s) selected from Substituent group α", it is preferably a 5- to 7- membered heteroaryl group containing from 1 to 3 sulfur atoms, oxygen atoms and/or nitrogen atoms substituted by from 1 to 3 groups selected from Substituent group α, more preferably a 5-to 7-membered heteroaryl group containing from 1 to 3 sulfur atoms, oxygen atoms and/or nitrogen atoms substituted by 1 or 2 groups selected from Substituent group α, still more preferably a 5- to 7-membered heteroaryl group containing from 1 to 3 sulfur atoms, oxygen atoms and/or nitrogen atoms substituted by 1 or 2 groups selected from the group consisting of fluorine atoms, chlorine atoms, methyl, ethyl, methoxy and ethoxy groups.

The "amino protecting group" in the definition of R¹ is not particularly limited, provided that it is a group generally used as an amino protecting group in the field of organic synthesis chemistry and provided that it is an acyl type (including sulfonyl type) group, and can be, for example, a C₁-C₆ alkanoyl group such as a formyl, acetyl, propionyl, butyryl, isobutyryl, pentanoyl, pivaloyl, isovaleryl or hexanoyl group; a C₁-C₄ alkanoyl group substituted by halogen atom(s) or a C₁-C₄ alkoxy such as a chloroacetyl, dichloroacetyl, trichloroacetyl, trifluoroacetyl, 3-fluoropropionyl, 4,4-dichlorobutyryl, methoxyacetyl, butoxyacetyl, ethoxypropionyl or propoxybutyryl group; an unsaturated C₂-C₄ alkanoyl group such as an acryloyl, propioloyl, methacryloyl, crotonoyl or isocrotonoyl group; a C₆-C₁₀ arylcarbonyl group which may be substituted by halogen atom(s), C₁-C₄ alkyl, C₁-C₄ alkoxy, C₁-C₄ alkoxycarbonyl, C₆-C₁₀ aryl or nitro such as a benzoyl, 1-naphthoyl, 2-naphthoyl, 2-fluorobenzoyl, 2-bromobenzoyl, 2,4-dichlorobenzoyl, 6-chloro-1-naphthoyl, p-toluoyl, 4-propylbenzoyl, 4-tert-butylbenzoyl, 2,4,6-trimethylbenzoyl, 6-ethyl-1-naphthoyl, p-anisoyl, 4-propoxybenzoyl, 4-tert-butoxybenzoyl, 6-ethoxy-1-naphthoyl, 2-ethoxycarbonylbenzoyl, 4-tert-butoxycarbonylbenzoyl, 6-methoxycarbonyl-1-naphthoyl, 4-phenylbenzoyl, 4-phenyl-1-naphthoyl, 6-α-naphthylbenzoyl, 4-nitrobenzoyl, 2-nitrobenzoyl or 6-nitro-1-naphthoyl group; a C₁-C₄ alkoxycarbonyl group which may be substituted by halogen or tri-C₁-C₄ alkylsilyl such as a methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, chloromethoxycarbonyl, 2,2,2-trichloroethoxycarbonyl, 2-fluoropropoxycarbonyl, 2-bromo-1,1-dimethylethoxycarbonyl, 2,2-dibromo-1,1-dimethylethoxycarbonyl, triethylsilylmethoxycarbonyl, 2-(trimethylsilyl)ethoxycarbonyl, 4-tripropylsilylbutoxycarbonyl or 3-(tert-butyldimethylsilyl)propoxycarbonyl group; a C₂-C₅ alkenyloxycarbonyl group such as a vinyloxycarbonyl, allyloxycarbonyl, 1,3-butadienyloxycarbonyl or 2-pentenyloxycarbonyl group; or a C₇-C₁₅ aralkyloxycarbonyl group which may be substituted by methoxy or nitro such as a benzyloxycarbonyl, (1-phenyl)benzyloxycarbonyl, 1-naphthylmethyloxycarbonyl, 2-naphthylmethyloxycarbonyl, 9-anthrylmethyloxycarbonyl, 4-methoxybenzyloxycarbonyl or 4-nitrobenzyloxycarbonyl group; a lower alkanesulfonyl group such as a methanesulfonyl or ethanesulfonyl group; a halogeno lower alkanesulfonyl group such as a trifluoromethanesulfonyl group or pentafluoroethanesulfonyl group; or an arylsulfonyl group such as a benzenesulfonyl, p-toluenesulfonyl or 4-nitrobenzenesulfonyl group, preferably a C₁-C₄ alkanoyl, trifluoroacetyl, methoxyacetyl, benzoyl, 1-naphthoyl, 2-naphthoyl, anisoyl, nitrobenzoyl, C₁-C₄ alkoxycarbonyl, 2,2,2-trichloroethoxycarbonyl, triethylsilylmethoxycarbonyl, 2-(trimethylsilyl)ethoxycarbonyl, vinyloxycarbonyl, allyloxycarbonyl, benzyloxycarbonyl or nitrobenzyloxycarbonyl group, more preferably a formyl, acetyl, trifluoroacetyl, benzoyl, p-anisoyl, 4-nitrobenzoyl, methoxycarbonyl, ethoxycarbonyl, butoxycarbonyl, tert-butoxycarbonyl, benzyloxycarbonyl or 4-nitrobenzyloxycarbonyl group, particularly preferably a methoxycarbonyl, ethoxycarbonyl, tert-butoxycarbonyl, benzyloxycarbonyl or trifluoroacetyl group, most preferably an ethoxycarbonyl or tert-butoxycarbonyl group.

The "C₁-C₆ alkyl group" in the definition of Substituent group α can be a straight or branched alkyl group such as a methyl, ethyl, propyl, isopropyl, butyl, isobutyl, sec-butyl, tert-butyl, pentyl, isopentyl, 2-methylbutyl, neopentyl, 1,1-dimethylpropyl, 1-ethylpropyl, hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl or 2-ethylbutyl group; and, as for Substituent group α, it is preferably a C₁-C₄ straight or branched alkyl group, more preferably a methyl, ethyl, propyl, isopropyl or butyl group, particularly preferably a methyl, ethyl or propyl group.

The "C₁-C₆ alkoxy group" in the definition of Substituent group α is a group in which an oxygen atom is bonded to the above "C₁-C₆ alkyl group", preferably a C₁-C₄ straight or branched alkoxy group, more preferably a methoxy, ethoxy, propoxy, isopropoxy or butoxy group, particularly preferably a methoxy, ethoxy or propoxy group.

The "halogen atom" in the definition of Substituent group α and the halogen atom of "a phenyl group substituted by 1 or 2 halogen atoms" in the definition of R⁴ are a fluorine atom, a chlorine atom, a bromine atom or an iodine atom, preferably a fluorine atom or a chlorine atom.

The C₁-C₄ alkoxy group of "a phenyl group substituted by from 1 to 3 groups selected from hydroxyl groups, C₁-C₄ alkoxy group, C₁-C₄ halogenated alkyl groups and a tetrazolyl group" in the definition of R³ can be a straight or branched alkoxy group such as a methoxy, ethoxy, propoxy, isopropoxy or butoxy group, preferably a methoxy, ethoxy or propoxy group, more preferably a methoxy or ethoxy group, particularly preferably a methoxy group.

The C₁-C₄ halogenated alkyl group of "a phenyl group substituted by from 1 to 3 groups selected from hydroxyl groups, C₁-C₄ alkoxy groups, C₁-C₄ halogenated alkyl groups and a tetrazolyl group" in the definition of R³ is a group in which 1 or 2 or more hydrogen atoms of C₁-C₄ alkyl group are replaced with the above "halogen atoms", and it is preferably a trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl or 2,2-dibromoethyl group, more preferably a trifluoromethyl, trichloromethyl, difluoromethyl or fluoromethyl group, and particularly preferably a trifluoromethyl group.

Although there are no particular limitations on the "leaving group" in the definition of Y, provided that it is a leaving group that is used during nucleophilic substitution reactions, it can be, for example, a halogen atom such as chlorine, bromine or iodine; a lower alkoxycarbonyloxy group such as a methoxycarbonyloxy or ethoxycarbonyloxy group; a lower alkanesulfonyloxy group such as a methanesulfonyloxy or ethanesulfonyloxy group; a halogeno lower alkanesulfonyloxy group such as a trifluoromethanesulfonyloxy or pentafluoroethanesulfonyloxy group; or an arylsulfonyloxy group such as a benzenesulfonyloxy, p-toluenesulfonyloxy or 4-nitrobenzenesulfonyloxy group, more preferably a halogen atom, a halogeno lower alkanesulfonyloxy group or arylsulfonyloxy group, and even more preferably an arylsulfonyloxy group.

Ar is preferably a phenyl group which may be substituted by one or more group(s) selected from Substituent group α, more preferably a phenyl group or a phenyl group substituted by 1 or 2 groups selected from the group consisting of methyl, ethyl, methoxy, fluorine atom and chlorine atom, particularly preferably a phenyl group.

Substituent group α preferably consists of C₁-C₄ alkyl groups, C₁-C₄ alkoxy groups and halogen atoms, more preferably fluorine atoms, chlorine atoms, methyl, ethyl, methoxy and ethoxy groups.

R² is preferably a hydrogen atom, ethoxycarbonyl or tert-butoxycarbonyl, particularly preferably a hydrogen atom.

R³ is preferably a phenyl group substituted by from 1 to 3 groups selected from the group consisting of hydroxyl, methoxy, ethoxy, trifluoromethyl, trichloromethyl, difluoromethyl, fluoromethyl and tetrazolyl groups, more preferably a phenyl group substituted by from 1 to 3 groups selected from the group consisting of hydroxyl, methoxy, trifluoromethyl and tetrazolyl groups (for example, 3,5-bis(trifluoromethyl)phenyl, 3,4,5-trimethoxyphenyl, 3-hydroxy-4,5-dimethoxyphenyl, 4-hydroxy-3,5-dimethoxyphenyl or 2-methoxy-5-(1-tetrazolyl)phenyl, still more preferably a phenyl group substituted by from 1 to 3 groups selected from the group consisting of methoxy, trifluoromethyl and tetrazolyl groups (for example, 3,5-bis(trifluoromethyl)phenyl, 3,4,5-trimethoxyphenyl or 2-methoxy-5-(1-tetrazolyl)phenyl), particularly preferably 3,5-bis(trifluoromethyl)phenyl or 3,4,5-trimethoxyphenyl.

R⁴ is preferably a phenyl group substituted by one or two fluorine atoms or chlorine atoms, more preferably a phenyl group substituted by two fluorine atoms or chlorine atoms, still more preferably 3,4-difluorophenyl or 3,4-dichlorophenyl, particularly preferably 3,4-dichlorophenyl.

n is preferably 2.

Of the compounds of formula (1), a preferable compound is spiro[benzo[c]thiophene-1(3H),4'-piperidine] 2-oxide and a more preferable compound is (2S)-spiro[benzo[c]thiophene-1(3H),4'-piperidine] 2-oxide.

Of the compounds of formula (2), preferable compounds are
methyl spiro[benzo[c]thiophene-1(3H),4'-piperidine]-1'-carboxylate,
ethyl spiro[benzo[c]thiophene-1(3H),4'-piperidine]-1'-carboxylate,
tert-butyl spiro[benzo[c]thiophene-1(3H),4'-piperidine]-1'-carboxylate,
benzyl spiro[benzo[c]thiophene-1(3H),4'-piperidine]-1'-carboxylate, and
1'-trifluoroacetylspiro[benzo[c]thiophene-1(3H),4'-piperidine]; and more preferable compounds are ethyl spiro[benzo[c]thiophene-1(3H),4'-piperidine]-1'-carboxylate, and
tert-butyl spiro[benzo[c]thiophene-1(3H),4'-piperidine]-1'-carboxylate.

### [MODE FOR CARRYING OUT THE INVENTION]

The present invention is carried out by the following steps.

### <Oxidation of cyclic thiol>

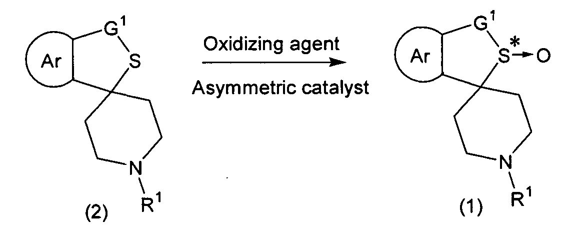

The oxidation of the compound of the above formula (2) is carried out by reacting it with an oxidizing agent in the presence of alcohol, water or a mixture of water and alcohol and in the presence of a complex of an optically active tartaric acid diester and a titanium (IV) alkoxide in an inert solvent.

The inert solvent to be used is not particularly limited, provided that it does not inhibit the reaction and provided that it dissolves the starting material, and can be, for example, aliphatic hydrocarbons such as hexane, heptane or petroleum ether; aromatic hydrocarbons such as benzene, toluene or xylene; ethers such as diethyl ether, diisopropyl ether, dibutyl ether, tert-butyl methyl ether, tetrahydrofuran or dioxane; esters such as methyl acetate, ethyl acetate, propyl acetate or butyl acetate; aliphatic halogenated hydrocarbons such as methylene chloride, chloroform, carbon tetrachloride or dichloroethane; aromatic halogenated hydrocarbons such as chlorobenzene, fluorobenzene, o-dichlorobenzene, m-dichlorobenzene, trichloromethylbenzene or trifluoromethylbenzene; amides such as N,N-dimethylformamide or N,N-dimethylacetamide; alcohols such as methanol, ethanol, propyl alcohol, isopropyl alcohol, butyl alcohol or tert-butyl alcohol; or nitriles such as acetonitrile, preferably aromatic hydrocarbons, esters, aliphatic halogenated hydrocarbons or aromatic halogenated hydrocarbons, more preferably the aliphatic halogenated hydrocarbons or aromatic halogenated hydrocarbons, still more preferably dichloroethane, chlorobenzene, o-dichlorobenzene, m-dichlorobenzene or trifluoromethylbenzene, most preferably chlorobenzene or o-dichlorobenzene.

The titanium (IV) alkoxide to be used for forming the complex can be, for example, titanium (IV) methoxide, titanium (IV) ethoxide, titanium (IV) propoxide or titanium (IV) isopropoxide, preferably titanium (IV) isopropoxide. The amount of titanium (IV) alkoxide to be used is preferably, relative to 1 equivalent of the compound of formula (2), from 0.01 to 0.4 equivalents, more preferably from 0.05 to 0.2 equivalents.

The optically active tartaric acid diester to be used for forming the complex can be, for example, an optically active tartaric acid diester such as dimethyl (+)- or (-)-tartrate, diethyl (+)- or (-)-tartrate, diisopropyl (+)- or (-)-tartrate, dibutyl (+)- or (-)-tartrate or di-tert-butyl (+)- or (-)-tartrate, preferably diethyl (+)- or (-)-tartrate or diisopropyl (+)- or (-)-tartrate, more preferably diisopropyl (+)- or (-)-tartrate. In the case where the compound of formula (1) having the S configuration is prepared, dimethyl (-)-tartrate, diethyl (-)-tartrate, diisopropyl (-)-tartrate, dibutyl (-)-tartrate or di-tert-butyl (-)-tartrate is preferably used, more preferably diethyl (-)-tartrate or diisopropyl (-)-tartrate is used, and particularly preferably diisopropyl (-)-tartrate is used. The amount of optically active diol to be used is preferably, relative to 1 equivalent of titanium (IV) alkoxide, from 1 to 10 equivalents, more preferably from 2 to 5 equivalents, particularly preferably about 4 equivalents.

The complex of the optically active tartaric acid diester and titanium (IV) alkoxide is used in the presence of alcohol, water or a mixture of water and alcohol. The alcohol to be used here can be, for example, methanol, ethanol, propyl alcohol, isopropyl alcohol, butyl alcohol, tert-butyl alcohol or phenol, preferably methanol, ethanol, isopropyl alcohol or phenol, more preferably isopropyl alcohol. The amount of alcohol to be used is preferably, relative to 1 equivalent of titanium (IV) alkoxide, from 0.5 to 100 equivalents, more preferably from 1 to 20 equivalents. The amount of water to be used is preferably, relative to 1 equivalent of titanium (IV) alkoxide, from 0.01 to 4 equivalents, more preferably from 0.02 to 2 equivalents.

The above complex is preferably used in the presence of alcohol (most preferably isopropyl alcohol) and it is further desirable that from 0.01 to 4 equivalents (preferably from 0.02 to 2 equivalents, more preferably from 0.03 to 1.5 equivalents) of water relative to 1 equivalent of titanium (IV) alkoxide is present.

In the embodiment in which the complex of the optically active tartaric acid diester and titanium (IV) alkoxide is used, [1] after the optically active tartaric acid diester is added to the inert solvent, the titanium (IV) alkoxide is added thereto, then the alcohol, water or a mixture of water and alcohol is added thereto and the compound of formula (2) is finally added thereto; or [2] after the optically active tartaric acid diester is added to the inert solvent containing the compound of formula (2), the titanium (IV) alkoxide is added thereto and then the alcohol, water or a mixture of water and alcohol is added thereto. The temperature in the case where the present operation is carried out is from 0 to 100°C, preferably from 15 to 30°C.

The oxidizing agent to be used can be cumene hydroperoxide or isopropylcumyl hydroperoxide, preferably cumene hydroperoxide. The amount of oxidizing agent to be used is preferably, relative to 1 equivalent of the compound of formula (2), from 0.5 to 10 equivalents, more preferably from 1 to 1.5 equivalents.

The temperature in the case where the oxidizing agent is allowed to be reacted is from -80 to 100°C, preferably from -20 to -5°C. The reaction time in the case where the oxidizing agent is allowed to be reacted varies depending on the reaction temperature, etc. but it is normally from 10 minutes to 20 hours, preferably from 3 hours to 10 hours.

After the reaction, the desired compound is recovered from the reaction mixture in accordance with ordinary methods.

For example, after suitably neutralizing the reaction mixture and removing any insoluble matter by filtration if present, water is added followed by extracting with an immiscible organic solvent like toluene, washing with water and so forth, drying the extract with anhydrous magnesium sulfate and so forth and distilling off the solvent to obtain the desired compound.

The resulting compound can be separated and purified by ordinary methods such as silica gel column chromatography as necessary.

### <Optical resolution>

The enantiomeric excess of the desired compound recovered from the reaction mixture can be enhanced by separation, purification or the like by recrystallization or by using an optically active column [for example, CHRALCEL (trade name, manufactured by Daicel Chemical Industries, LTD.)].

For example, in the case of purifying it by recrystallization, the enantiomeric excess can be enhanced by carrying out the recrystallization without removing the protecting group of the amino group; or (a) removing the amino protecting group and then (b) carrying out the optical resolution by the diastereomer method.

### (a) Removal of the amino protecting group

The removal of the amino protecting group is carried out according to well known methods and, for example, the amino protecting group can be removed by treating it with an acid or a base in an inert solvent.

The solvent to be used here is not particularly limited, and provided that it does not inhibit the reaction and provided that it dissolves the starting material to a certain degree, and can be, for example, aliphatic hydrocarbons such as hexane, heptane, ligroin or petroleum ether; aromatic hydrocarbons such as benzene, toluene or xylene; halogenated hydrocarbons such as dichloromethane, chloroform, carbon tetrachloride, dichloroethane, chlorobenzene, o-dichlorobenzene, m-dichlorobenzene, fluorobenzene, trichloromethylbenzene or trifluoromethylbenzene; ethers such as diethyl ether, diisopropyl ether, tetrahydrofuran, dioxane, 1,2-dimethoxyethane or diethylene glycol dimethyl ether; esters such as methyl acetate or ethyl acetate; alcohols such as methanol, ethanol, propyl alcohol, isopropyl alcohol or butyl alcohol; amides such as formamide, N,N-dimethylformamide, N,N-dimethylacetamide or hexamethylphosphorotriamide; sulfoxides or sulfones such as dimethyl sulfoxide or sulforane; aliphatic acids such as formic acid or acetic acid; or water or a mixed solvent of water and the above solvents, preferably halogenated hydrocarbons, ethers, alcohols, aliphatic acids or a mixed solvent of water and the above solvents, more preferably halogenated hydrocarbons (particularly chlorobenzene, o-dichlorobenzene, m-dichlorobenzene or trifluoromethylbenzene), ethers (particularly tetrahydrofuran or dioxane), aliphatic acids (particularly acetic acid), alcohols (particularly methanol or ethanol) or a mixed solvent of water and the above solvents.

The acid to be used can be, for example, hydrogen chloride, hydrochloric acid, sulfuric acid, phosphoric acid, hydrogen bromide, hydrobromic acid or trifluoroacetic acid, preferably hydrochloric acid, sulfuric acid, hydrobromic acid or trifluoroacetic acid.

The base to be used can be, for example, alkali metal carbonates such as sodium carbonate, potassium carbonate or lithium carbonate; alkali metal bicarbonates such as sodium hydrogencarbonate, potassium hydrogencarbonate or lithium hydrogencarbonate; alkali metal hydrides such as lithium hydride, sodium hydride or potassium hydride; alkali metal hydroxides such as sodium hydroxide, potassium hydroxide or lithium hydroxide; alkali metal alkoxides such as sodium methoxide, sodium ethoxide, potassium tert-butoxide or lithium methoxide; alkali metal thioalkoxides such as sodium thiomethoxide or sodium thioethoxide; or organic bases such as hydrazine, methylamine, dimethylamine, ethylamine, triethylamine, tributylamine, diisopropylethylamine, N-methylmorpholine, pyridine, 4-dimethylaminopyridine, N,N-dimethylaniline, N,N-diethylaniline, 1,5-diazabicyclo[4.3.0]non-5-ene, 1,4-diazabicyclo[2.2.2]octane (DABCO) or 1,8-diazabicyclo[5.4.0]undec-7-ene (DBU), preferably alkali metal carbonates (particularly sodium carbonate or potassium carbonate), alkali metal hydroxides (particularly sodium hydroxide or potassium hydroxide), alkali metal alkoxides (particularly sodium methoxide, sodium ethoxide or potassium tert-butoxide) or organic bases (particularly hydrazine or methylamine).

While the reaction temperature varies depending on the raw material compound, solvent or acid or base used, it is normally from -10°C to 150°C, preferably from 0°C to 100°C.

While the reaction time varies depending on the raw material compound, solvent or acid or base used, it is normally from 5 minutes to 48 hours, preferably from 10 minutes to 15 hours.

### (b) Optical resolution by diastereomer method

The optical resolution by the diastereomer method is carried out, for example, by recrystallizing from aliphatic hydrocarbons such as hexane, heptane or petroleum ether; aromatic hydrocarbons such as benzene, toluene or xylene; ethers such as diethyl ether, diisopropyl ether, dibutyl ether, tert-butyl methyl ether, tetrahydrofuran or dioxane; esters such as methyl acetate, ethyl acetate, propyl acetate or butyl acetate; aliphatic halogenated hydrocarbons such as methylene chloride, chloroform, carbon tetrachloride or dichloroethane; alcohols such as methanol, ethanol, propyl alcohol, isopropyl alcohol, butyl alcohol or tert-butyl alcohol; nitriles such as acetonitrile; ketones such as acetone; or a solvent mixture of a water-soluble solvent such as alcohols, tetrahydrofuran, dioxane, acetonitrile or acetone with water, using an optical resolution agent.

The optical resolution agent to be used is not particularly limited, provided that it is normally used as an optical resolution agent, and it can be, for example, optically active sulfonic acids such as (+)- or (-)-camphor-10-sulfonic acid; or optically active carboxylic acids such as (+)- or (-)-tartaric acid, diacetyl (+)- or (-)-tartaric acid, dibenzoyl (+)- or (-)-tartaric acid, (+)- or (-)-mandelic acid or (+)- or (-)-malic acid, preferably optically active carboxylic acids, more preferably (+)- or (-)-mandelic acid.

In the case of obtaining a compound of formula (1) having the S configuration, (+)-tartaric acid, dibenzoyl (+)-tartaric acid, (-)-camphor-10-sulfonic acid or (+)-mandelic acid is preferably used, more preferably (+)-mandelic acid is used.

The compound (2), i.e., the starting material in the process of the present invention, is disclosed, for example, in US6159967 and US6362179.

The compound of formula (1) can be easily led to a neurokinin receptor antagonist by the method disclosed in, for example, WO95/28389 and USP6159967. In more detail, the neurokinin receptor antagonist can be prepared by carrying out the reaction according to the following process: 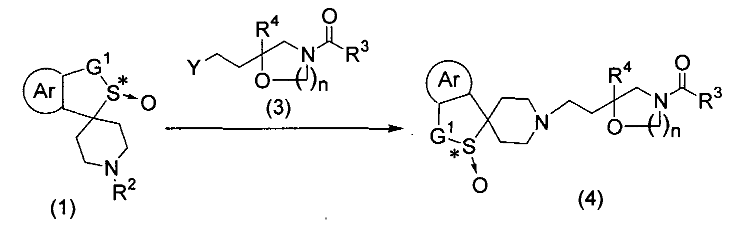 (wherein Ar, G¹, R², R³, R⁴, Y, n and * have the same meanings as defined above).

The present step is a step to prepare the compound (4) by reacting the compound (1) with the compound (3). In the case where R² is an amino protecting group, R² is firstly removed and then the resulting compound is reacted with the compound (3).

The removal of the amino protecting group is carried out according to well known methods and the amino protecting group can be removed, for example, by treating with an acid or a base in an inert solvent.

The solvent to be used is not particularly limited, provided that it does not inhibit the reaction and provided that it dissolves the starting material to a certain degree, and can be, for example, aliphatic hydrocarbons such as hexane, heptane, ligroin or petroleum ether; aromatic hydrocarbons such as benzene, toluene or xylene; halogenated hydrocarbons such as dichloromethane, chloroform, carbon tetrachloride, dichloroethane, chlorobenzene, o-dichlorobenzene, m-dichlorobenzene, fluorobenzene, trichloromethylbenzene or trifluoromethylbenzene; ethers such as diethyl ether, diisopropyl ether, tetrahydrofuran, dioxane, 1,2-dimethoxyethane or diethylene glycol dimethyl ether; esters such as methyl acetate or ethyl acetate; alcohols such as methanol, ethanol, propyl alcohol, isopropyl alcohol or butyl alcohol; amides such as formamide, N,N-dimethylformamide, N,N-dimethylacetamide or hexamethylphosphorotriamide; sulfoxides or sulfones such as dimethyl sulfoxide or sulforane; aliphatic acids such as formic acid or acetic acid; or water or a mixed solvent of water and the above solvents, preferably halogenated hydrocarbons, ethers, alcohols, aliphatic acids or a mixed solvent of water and the above solvents, more preferably halogenated hydrocarbons (particularly chlorobenzene, o-dichlorobenzene, m-dichlorobenzene or trifluoromethylbenzene), ethers (particularly tetrahydrofuran or dioxane), aliphatic acids (particularly acetic acid), alcohols (particularly methanol or ethanol) or a mixed solvent of water and the above solvents.

The acid to be used can be, for example, hydrogen chloride, hydrochloric acid, sulfuric acid, phosphoric acid, hydrogen bromide, hydrobromic acid or trifluoroacetic acid, preferably hydrochloric acid, sulfuric acid, hydrobromic acid or trifluoroacetic acid.

The base to be used can be, for example, alkali metal carbonates such as sodium carbonate, potassium carbonate or lithium carbonate; alkali metal bicarbonates such as sodium hydrogencarbonate, potassium hydrogencarbonate or lithium hydrogencarbonate; alkali metal hydrides such as lithium hydride, sodium hydride or potassium hydride; alkali metal hydroxides such as sodium hydroxide, potassium hydroxide or lithium hydroxide; alkali metal alkoxides such as sodium methoxide, sodium ethoxide, potassium tert-butoxide or lithium methoxide; alkali metal thioalkoxides such as sodium thiomethoxide or sodium thioethoxide;,or organic bases such as hydrazine, methylamine, dimethylamine, ethylamine, triethylamine, tributylamine, diisopropylethylamine, N-methylmorpholine, pyridine, 4-dimethylaminopyridine, N,N-dimethylaniline, N,N-diethylaniline, 1,5-diazabicyclo[4.3.0]non-5-ene, 1,4-diazabicyclo[2.2.2]octane (DABCO) or 1,8-diazabicyclo[5.4.0]undec-7-ene (DBU), preferably alkali metal carbonates (particularly sodium carbonate or potassium carbonate), alkali metal hydroxides (particularly sodium hydroxide or potassium hydroxide), alkali metal alkoxides (particularly sodium methoxide, sodium ethoxide or potassium tert-butoxide) or organic bases (particularly hydrazine or methylamine).

While the reaction temperature varies depending on the raw material compound, the solvent or the acid or base used, it is normally from -10°C to 150°C, preferably from 0°C to 100°C.

While the reaction time varies depending on the raw material compound, the solvent or the acid or base used, it is normally from 5 minutes to 48 hours, preferably from 10 minutes to 15 hours.

The reaction of the compound (1) in which R² is a hydrogen atom with the compound (3) is carried out in the presence of a base in an inert solvent.

The inert solvent to be used is not particularly limited, provided that it does not inhibit the reaction and provided that it dissolves the starting material to a certain degree, and can be, for example, aliphatic hydrocarbons such as hexane, heptane, ligroin or petroleum ether; aromatic hydrocarbons such as benzene, toluene or xylene; halogenated hydrocarbons such as dichloromethane, chloroform, carbon tetrachloride, dichloroethane, chlorobenzene or dichlorobenzene; esters such as ethyl formate, ethyl acetate, propyl acetate, butyl acetate or diethyl carbonate; ethers such as diethyl ether, diisopropyl ether, tetrahydrofuran, dioxane, 1,2-dimethoxyethane or diethylene glycol dimethyl ether; ketones such as acetone, ethyl methyl ketone, isobutyl methyl ketone, isophorone or cyclohexanone; nitro compounds such as nitroethane or nitrobenzene; nitriles such as acetonitrile or isobutyronitrile; amides such as formamide, N,N-dimethylformamide, N,N-dimethylacetamide, N-methyl-2-pyrrolidone, N-methylpyrrolidinone or hexamethylphosphorotriamide; or sulfoxides such as dimethyl sulfoxide or sulforane, preferably amides, ethers or nitriles, particularly preferably nitriles.

The base to be used is not particularly limited, provided that it is used as a base in a normal reaction, and can be, for example, alkali metal carbonates such as sodium carbonate, potassium carbonate or lithium carbonate; alkali earth metal carbonates such as calcium carbonate or barium carbonate; alkali metal hydrogencarbonates such as sodium hydrogencarbonate, potassium hydrogencarbonate or lithium hydrogencarbonate; alkali metal hydrides such as lithium hydride, sodium hydride or potassium hydride; alkali metal hydroxides such as sodium hydroxide, potassium hydroxide or lithium hydroxide; or alkali earth metal hydroxides such as calcium hydroxide or barium hydroxide;: or organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-dimethylaminopyridine, 2,6-di(tert-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU), preferably inorganic bases, most preferably alkali metal hydrogencarbonates. Further, for the purpose of promoting the reaction, it is also useful to add a catalytic amount of alkali metal iodides such as potassium iodide or sodium iodide.

The reaction temperature can be, for example, from 0°C to 150°C, preferably from 20°C to 120°C.

While the reaction time varies mainly depending on the reaction temperature, the raw material compound, the reaction reagent or the kind of the inert solvent used, it can be from 30 minutes to 48 hours, preferably from 1 hour to 12 hours.

After the reaction, the desired compound is recovered from the reaction mixture in accordance with ordinary methods.

For example, water is added to the reaction mixture followed by extracting with an immiscible organic solvent like toluene, washing with water and so forth, drying the extract with anhydrous magnesium sulfate and so forth and distilling off the solvent to obtain the desired compound.

The resulting compound can be separated and purified by ordinary methods such as silica gel column chromatography as necessary.

Further, the compound (4) can be easily led to a pharmaceutically acceptable salt, if desired, by treating it according to ordinary methods using an acid (said acid can be, for example, inorganic acids such as hydrogen chloride, sulfuric acid or phosphoric acid; or organic acids such as acetic acid, fumaric acid or succinic acid, preferably hydrogen chloride or fumaric acid).

### [BEST MODE FOR CARRYING OUT THE INVENTION]

The present invention will be more specifically explained below by referring to Examples but the present invention is not limited to these.

It should be noted that both of the enantiomeric excess and the diastereomeric excess in the respective Examples are values based on an analysis by high performance liquid chromatography (HPLC). The enantiomeric excess was analyzed under the condition of the following "HPLC condition (1)" and the diastereomeric excess was analyzed under the condition of the following "HPLC condition (2)". Further, HPLC for analyzing the composition of the reaction mixture was carried out under the condition of the following "HPLC condition (3)".

### <HPLC condition (1)>

Column: CHRALCEL OD (trade name, manufactured by Daicel Chemical Industries, LTD.) 4.6φ × 250 mm
Mobile phase: Hexane:EtOH = 90:10
Column temperature: 40°C
Detection: UV (220 nm)
Flow rate: 1 ml/min

### <HPLC condition (2)>

Column: CHRALCEL OD (trade name, manufactured by Daicel Chemical Industries, LTD.) 4.6φ × 250 mm
Mobile phase: Hexane:EtOH = 85:15
Column temperature: 40°C
Detection: UV (220 nm)
Flow rate: 1 ml/min

### <HPLC condition (3)>

Column: L-column ODS (trade name, manufactured by
Kagaku Busshitu Hyoka Kenkyu Kiko (Chemicals Evaluation and Research Institute, Japan) 4.6φ × 250 mm
Mobile phase: CH₃CN:0.01M Na₂HPO₄ = 40:60 → 75:25 (gradient)
Column temperature: 40°C
Detection: UV (220 nm)
Flow rate: 1 ml/min

| Gradient condition | |
|---|---|
| Time (minute) | CH₃CN concentration (%) |
| 5.00 | 40 |
| 15.00 | 75 |
| 26.00 | 75 |
| 26.01 | 40 |
| 30.00 | Stop |

### [Example 1]

### tert-Butyl (2S)-spiro[benzo[c]thiophene-1(3H),4'-piperidine]-1'-carboxylate 2-oxide

Under a nitrogen atmosphere, 0.99 ml (3.23 mmol) of titanium (IV) isopropoxide was added dropwise to a mixed solution of 1.35 ml (6.45 mmol) of diisopropyl (-)-tartrate and 40 ml of chlorobenzene at room temperature. After this solution was stirred at room temperature for approximately 20 minutes, 0.25 ml (3.22 mmol) of isopropyl alcohol was added thereto at room temperature and the mixture was further stirred for approximately 10 minutes. After cooling it to -10°C or lower, 5.00 g (16.2 mmol) of tert-butyl spiro[benzo[c]thiophene-1(3H),4'-piperidine]-1'-carboxylate were added thereto. A solution of 2.95 ml (17.7 mmol) of cumene hydroperoxide in 10 ml of chlorobenzene was added dropwise to the mixture at -10°C or lower and the mixture was stirred at -10°C for 5 hours. When the reaction mixture was analyzed by HPLC, the mixture was constituted by 86.7 % tert-butyl (2S)-spiro[benzo[c]thiophene-1(3H),4'-piperidine]-1'-carboxylate 2-oxide, 5.5% tert-butyl spiro[benzo[c]thiophene-1(3H),4'-piperidine]-1'-carboxylate 2,2-dioxide and 1.0% tert-butyl spiro[benzo[c]thiophene-1(3H),4'-piperidine]-1'-carboxylate and the enantiomeric excess of the title compound was 84.9%.

25 ml of 5% aqueous solution of sodium pyrosulfite were added to the reaction mixture and the mixture was stirred at 0 to 5°C for 30 minutes. Any insoluble matter was filtered off and after the separated organic layer was concentrated under reduced pressure, 50 ml of ethylcyclohexane were added and the mixture was stirred at room temperature for 1 hour and further at 0 to 5°C for 30 minutes. Further, 25 ml of ethylcyclohexane were added thereto and the mixture was stirred at 0 to 5°C for 30 minutes. After the precipitated crystals were collected by filtration, followed by drying at 50°C under reduced pressure for 15 hours to obtain tert-butyl (2S)-spiro[benzo[c]thiophene-1(3H),4'-piperidine]-1'-carboxylate 2-oxide having crude yield: 49.0% (2.58 g) and the enantiomeric excess: 99.1% as white crystals.
¹H-NMR (400 MHz, CDCl₃) δ ppm: 1.50 (s, 9H), 1.60-1.66 (m, 1H), 1.86-1.94 (m, 1H), 2.16-2.24 (m, 1H), 2.40-2.48 (m, 1H), 3.10-3.30 (m, 2H), 4.05 (d, *J*=16.8 Hz, 1H), 4.10-4.30 (m, 2H), 4.37 (d, *J*=16.8 Hz, 1H), 7.20-7.40 (m, 4H).

### [Example 2]

### (S)-(+)-Mandelic acid salt of (2S)-spiro[benzo[c]thiophene-1(3H),4'-piperidine] 2-oxide

Under a nitrogen atmosphere, 74.4 g (243.5 mmol) of tert-butyl spiro[benzo[c]thiophene-1(3H),4'-piperidine]-1'-carboxylate and 525 ml of chlorobenzene were mixed and 22.8 g (97.4 mmol) of diisopropyl (-)-tartrate were further added thereto at room temperature. Then, 7.47 ml (24.3 mmol) of titanium (IV) isopropoxide were added dropwise thereto at room temperature and the mixture was stirred at the same temperature for approximately 20 minutes. 7.48 ml (97.3 mmol) of isopropyl alcohol were added thereto at room temperature and the mixture was further stirred for approximately 20 minutes. After cooling it to -10°C or lower, a solution of 55.6 g (292.2 mmol) of cumene hydroperoxide in 150 ml of chlorobenzene was added dropwise thereto at -10°C or lower and the mixture was stirred at -10°C for 5 hours. When the reaction mixture was analyzed by HPLC, the mixture was constituted by 87.3% tert-butyl (2S)-spiro[benzo[c]thiophene-1(3H),4'-piperidine]-1'-carboxylate 2-oxide, 3.3% tert-butyl spiro[benzo[c]thiophene-1(3H),4'-piperidine]-1'-carboxylate 2,2-dioxide and 0.6% tert-butyl spiro[benzo[c]thiophene-1(3H),4'-piperidine]-1'-carboxylate and the enantiomeric excess of the title compound was 87.2%.

194.5 ml of 10% aqueous solution of sodium pyrosulfite were added dropwise to the reaction mixture and the mixture was stirred at 0 to 5°C for 30 minutes. 122 ml (1.46 mol) of conc. hydrochloric acid were added to the mixture and the mixture was stirred at 50°C for 4 hours. The organic layer was separated and 375 ml of butanol and 273 g of 25% aqueous solution of sodium hydroxide were added to the aqueous layer to extract it. The aqueous layer was separated and extracted with 375 ml of butanol. After the organic layer was combined, it was concentrated under reduced pressure until the amount of liquid became 150 ml. Any insoluble matter was removed by filtration and 600 ml of isopropyl alcohol and 18.6 ml of water were added thereto, followed by addition of 34.8 g (226.7 mmol) of (+)-mandelic acid. After the mixture was stirred at 55°C for 30 minutes, it was stirred at room temperature for 30 minutes and was further stirred at 0 to 5°C for 1 hour. The precipitated crystals were collected by filtration and dried at 60°C under reduced pressure for 15 hours to obtain the title compound [yield: 82.1% (75.1 g), diastereomeric excess: 99.3%] as white crystals.
¹H-NMR (400 MHz, CD₃OD) δ ppm: 1.96 (m, 1H), 2.14 (ddd, *J*=15.9, 12.9, and 3.4 Hz, 1H), 2.44 (m, 1H), 2.55 (ddd, *J*=15.9, 13.2, 4.4 Hz, 1H), 3.21 (ddd, *J*=13.2, 13.2, 3.4 Hz, 1H), 3.28 (ddd, *J*=13.7, 12.9, 3.4 Hz, 1H), 3.45 (m, 1H), 3.51 (m, 1H), 4.14 (d, *J*=17.1 Hz, 1H), 4.65 (d, *J*=17.1 Hz, 1H), 4.90 (s, 1H), 7.18-7.50 (m, 9H).

### [Example 3]

### Methyl (2S)-spiro[benzo[c]thiophene-1(3H),4'-piperidine]-1'-carboxylate 2-oxide

Under a nitrogen atmosphere, 0.198 ml (0.64 mmol) of titanium (IV) isopropoxide was added dropwise to a mixed solution of 0.27 ml (1.29 mmol) of diisopropyl (-)-tartrate and 9 ml of o-dichlorobenzene at room temperature. After the solution was stirred at room temperature for approximately 20 minutes, 49.5 µl (0.64 mmol) of isopropyl alcohol were added thereto at room temperature and the mixture was further stirred for approximately 10 minutes. After cooling it to -10°C or lower, 0.85 g (3.22 mmol) of methyl spiro[benzo[c]thiophene-1(3H),4'-piperidine]-1'-carboxylate was added thereto. Then, a solution of 0.59 ml (3.54 mmol) of cumene hydroperoxide in 2 ml of o-dichlorobenzene was added dropwise thereto at -10°C or lower. After the mixture was stirred at -10°C for 5 hours, the reaction mixture was analyzed by HPLC, and the mixture was constituted by 84.7% methyl (2S)-spiro[benzo[c]thiophene-1(3H),4'-piperidine]-1'-carboxylate 2-oxide, 5.9% methyl spiro[benzo[c]thiophene-1(3H),4'-piperidine]-1'-carboxylate 2,2-dioxide and 1.3% methyl spiro[benzo[c]thiophene-1(3H),4'-piperidine]-1'-carboxylate and the enantiomeric excess of the title compound was 85.6%.
¹H-NMR (400 MHz, CDCl₃) δ ppm: 1.55-1.68 (m, 1H), 1.82-2.00 (m, 1H), 2.13-2.28 (m, 1H), 2.39-2.51 (m, 1H), 3.15-3.35 (m, 2H), 3.76 (s, 3H), 4.06 (d, *J*=16.8 Hz, 1H), 4.10-4.45 (m, 2H), 4.38 (d, *J*=16.8 Hz, 1H), 7.17-7.40 (m, 4H).

### [Example 4]

### Ethyl (2S)-spiro[benzo[c]thiophene-1(3H),4'-piperidine]-1'-carboxylate 2-oxide

Under a nitrogen atmosphere, 0.198 ml (0.64 mmol) of titanium (IV) isopropoxide was added dropwise to a mixed solution of 0.27 ml (1.29 mmol) of diisopropyl (-)-tartrate and 9 ml of o-dichlorobenzene at room temperature. After the solution was stirred at room temperature for approximately 20 minutes, 49.5 µl (0.64 mmol) of isopropyl alcohol were added thereto at room temperature and the mixture was further stirred for approximately 10 minutes. After cooling it to -10°C or lower, 0.89 g (3.22 mmol) of ethyl spiro[benzo[c]thiophene-1(3H),4'-piperidine]-1'-carboxylate was added thereto. Then, a solution of 0.59 ml (3.54 mmol) of cumene hydroperoxide in 2 ml of o-dichlorobenzene was added dropwise thereto at -10°C or lower. After the mixture was stirred at -10°C for 5 hours, the reaction mixture was analyzed by HPLC, and the mixture was constituted by 88.2% ethyl (2S)-spiro[benzo[c]thiophene-1(3H),4'-piperidine]-1'-carboxylate 2-oxide and 5.2% ethyl spiro[benzo[c]thiophene-1(3H),4'-piperidine]-1'-carboxylate 2,2-dioxide and the enantiomeric excess of the title compound was 83.3%.
¹H-NMR (400 MHz, CDCl₃) δ ppm: 1.30 (t, *J*=7.1 Hz, 3H), 1.55-1.65 (m, 1H), 1.83-1.98 (m, 1H), 2.15-2.28 (m, 1H), 2.40-2.50 (m, 1H), 3.15-3.35 (m, 2H), 4.06 (d, *J*=16.6 Hz, 1H), 4.20 (q, *J*=7.1 Hz, 2H), 4.10-4.45 (m, 2H), 4.38 (d, *J*=16.6 Hz, 1H), 7.20-7.40 (m, 4H) .

### [Example 5]

### Benzyl (2S)-spiro[benzo[c]thiophene-1(3H),4'-piperidine]-1'-carboxylate 2-oxide

Under a nitrogen atmosphere, 0.198 ml (0.64 mmol) of titanium (IV) isopropoxide was added dropwise to a mixed solution of 0.27 ml (1.29 mmol) of diisopropyl (-)-tartrate and 9 ml of o-dichlorobenzene at room temperature. After the solution was stirred at room temperature for approximately 20 minutes, 49.5 µl (0.64 mmol) of isopropyl alcohol were added thereto at room temperature and the mixture was further stirred for approximately 10 minutes. After cooling it to -10°C or lower, 1.10 g (3.24 mmol) of benzyl spiro[benzo[c]thiophene-1(3H),4'-piperidine]-1'-carboxylate were added thereto. Then, a solution of 0.59 ml (3.54 mmol) of cumene hydroperoxide in 2 ml of o-dichlorobenzene was added dropwise thereto at -10°C or lower. After the mixture was stirred at -10°C for 5 hours, the reaction mixture was analyzed by HPLC, and the mixture was constituted by 84.6% benzyl (2S)-spiro[benzo[c]thiophene-1(3H),4'-piperidine]-1'-carboxylate 2-oxide, 6.1% benzyl spiro[benzo[c]thiophene-1(3H),4'-piperidine]-1'-carboxylate 2,2-dioxide and 3.4% benzyl spiro[benzo[c]thiophene-1(3H),4'-piperidine]-1'-carboxylate and the enantiomeric excess of the title compound was 85.0%.
¹H-NMR (400 MHz, CDCl₃) δ ppm: 1.50-1.70 (m, 1H), 1.83-1.99 (m, 1H), 2.13-2.32 (m, 1H), 2.38-2.53 (m, 1H), 3.12-3.45 (m, 2H), 4.06 (d, J=16.8 Hz, 1H), 4.10-4.50 (m, 2H), 4.37 (d, J=16.8 Hz, 1H),5.19 (s, 2H), 7.15-7.50 (m, 9H).

### [Example 6]

### (2S)-1'-Trifluoroacetyl-spiro[benzo[c]thiophene-1(3H),4'-piperidine] 2-oxide

Under a nitrogen atmosphere, 0.198 ml (0.64 mmol) of titanium (IV) isopropoxide was added dropwise to a mixed solution of 0.27 ml (1.29 mmol) of diisopropyl (-)-tartrate and 9 ml of o-dichlorobenzene at room temperature. After the solution was stirred at room temperature for approximately 20 minutes, 49.5 µl (0.64 mmol) of isopropyl alcohol were added thereto at room temperature and the mixture was further stirred for approximately 10 minutes. After cooling it to -10°C or lower, 0.98 g (3.24 mmol) of 1'-trifluoroacetyl-spiro[benzo[c]thiophene-1(3H),4'-piperidine] was added thereto. Then, a solution of 0.59 ml (3.54 mmol) of cumene hydroperoxide in 2 ml of o-dichlorobenzene was added dropwise thereto at -10°C or lower. After the mixture was stirred at -10°C for 5 hours, the reaction mixture was analyzed by HPLC, and the mixture was constituted by 82.9% (2S)-1'-trifluoroacetyl-spiro[benzo[c]thiophene-1(3H),4'-piperidine] 2-oxide and 13.2% 1'-trifluoroacetyl-spiro[benzo[c]thiophene-1(3H),4'-piperidine] 2,2-dioxide and the enantiomeric excess of the title compound was 76.9%.
¹H-NMR (400 MHz, CDCl₃) δ ppm: 1.72-1.82 (m, 1H), 1.95-2.09 (m, 1H), 2.18-2.32 (m, 1H), 2.55-2.66 (m, 1H), 3.20-3.32 (m, 1H), 3.58-3.70 (m, 1H), 4.10 (d, *J*=14.4 Hz, 1H), 4.14 (d, *J*=14.4 Hz, 1H), 4.38-4.48 (m, 1H), 4.60-4.73 (m, 1H), 7.15-7.45 (m, 4H).

### [Example 7]

### tert-Butyl (2S)-spiro[benzo[c]thiophene-1(3H),4'-piperidine]-1'-carboxylate 2-oxide

Under a nitrogen atmosphere, 9.70 g (31.8 mmol) of tert-butyl spiro[benzo[c]thiophene-1(3H),4'-piperidine]-1'-carboxylate and 70 ml of chlorobenzene were mixed and 2.66 ml (12.7 mmol) of diisopropyl (-)-tartrate and 0.017 ml (0.96 mmol) of water were further added thereto at room temperature. Then, 0.975 ml (3.18 mmol) of titanium (IV) isopropoxide was added dropwise thereto at room temperature and the mixture was stirred at the same temperature for approximately 20 minutes. 0.98 ml (12.7 mmol) of isopropyl alcohol was added thereto at room temperature and the mixture was further stirred for approximately 20 minutes. After cooling it to -10°C or lower, a solution of 7.25 g (38.1 mmol) of cumene hydroperoxide in 18 ml of chlorobenzene was added dropwise thereto at -10°C or lower and the mixture was stirred at -10°C for 4 hours. The reaction mixture was analyzed by HPLC, and the mixture was constituted by 87.0% tert-butyl (2S)-spiro[benzo[c]thiophene-1(3H),4'-piperidine]-1'-carboxylate 2-oxide, 3.5% tert-butyl spiro[benzo[c]thiophene-1(3H),4'-piperidine]-1'-carboxylate 2,2-dioxide and 0.7% tert-butyl spiro[benzo[c]thiophene-1(3H),4'-piperidine)-1'-carboxylate and the enantiomeric excess of the title compound was 88.9%.

### [Example 8]

### tert-Butyl (2S)-spiro[benzo[c]thiophene-1(3H),4'-piperidine]-1'-carboxylate 2-oxide

Under a nitrogen atmosphere, 4.85 g (15.9 mmol) of tert-butyl spiro[benzo[c]thiophene-1(3H),4'-piperidine]-1'-carboxylate and 35 ml of chlorobenzene were mixed and 2.66 ml (12.7 mmol) of diisopropyl (-)-tartrate were further added thereto at room temperature. Then, 0.49 ml (1.59 mmol) of titanium (IV) isopropoxide was added dropwise thereto at room temperature and the mixture was stirred at the same temperature for approximately 20 minutes. 0.49 ml (6.35 mmol) of isopropyl alcohol was added thereto at room temperature and the mixture was further stirred for approximately 20 minutes. After cooling it to -10°C or lower, a solution of 3.30 g (19.1 mmol) of cumene hydroperoxide in 8.5 ml of chlorobenzene was added dropwise thereto at -10°C or lower and the mixture was stirred at -10°C for 6 hours. The reaction mixture was analyzed by HPLC, and the mixture was constituted by 87.7% tert-butyl (2S)-spiro[benzo[c]thiophene-1(3H),4'-piperidine]-1'-carboxylate 2-oxide, 2.7% tert-butyl spiro[benzo[c]thiophene-1(3H),4'-piperidine]-1'-carboxylate 2,2-dioxide and 0.8% tert-butyl spiro[benzo[c]thiophene-1(3H),4'-piperidine]-1'-carboxylate and the enantiomeric excess of the title compound was 90.2%.

### [Example 9]

### tert-Butyl (2S)-spiro[benzo[c]thiophene-1(3H),4'-piperidine]-1'-carboxylate 2-oxide

Under a nitrogen atmosphere, 4.85 g (15.9 mmol) of tert-butyl spiro[benzo[c]thiophene-1(3H),4'-piperidine]-1'-carboxylate and 35 ml of chlorobenzene were mixed and 1.33 ml (6.35 mmol) of diisopropyl (-)-tartrate were further added thereto at room temperature. Then, 0.49 ml (1.59 mmol) of titanium (IV) isopropoxide was added dropwise thereto at room temperature and the mixture was stirred at the same temperature for approximately 20 minutes. 0.49 ml (6.35 mmol) of isopropyl alcohol was added thereto at room temperature and the mixture was further stirred for approximately 20 minutes. After cooling it to -10°C or lower, a solution of 6.86 g (19.1 mmol) of isopropylcumyl hydroperoxide in 8.5 ml of chlorobenzene was added dropwise thereto at -10°C or lower and the mixture was stirred at -10°C for 5 hours. The reaction mixture was analyzed by HPLC, and the mixture was constituted by 86.7% tert-butyl (2S)-spiro[benzo[c]thiophene-1(3H),4'-piperidine]-1'-carboxylate 2-oxide, 3.9% tert-butyl spiro[benzo[c]thiophene-1(3H),4'-piperidine]-1'-carboxylate 2,2-dioxide and 1.4% tert-butyl spiro[benzo[c]thiophene-1(3H),4'-piperidine]-1'-carboxylate and the enantiomeric excess of the title compound was 88.4%.

### [Comparative example 1]

### tert-Butyl (2S)-spiro[benzo[c]thiophene-1(3H),4'-piperidine]-1'-carboxylate 2-oxide

### (Synthesis of the title compound in which p-mentyl hydroperoxide was used as an oxidizing agent)

Under a nitrogen atmosphere, 4.85 g (15.9 mmol) of tert-butyl spiro[benzo[c]thiophene-1(3H),4'-piperidine]-1'-carboxylate and 35 ml of chlorobenzene were mixed and 1.33 ml (6.35 mmol) of diisopropyl (-)-tartrate were further added thereto at room temperature. Then, 0.49 ml (1.59 mmol) of titanium (IV) isopropoxide was added dropwise thereto at room temperature and the mixture was stirred at the same temperature for approximately 20 minutes. 0.49 ml (6.35 mmol) of Isopropyl alcohol was added thereto at room temperature and the mixture was further stirred for approximately 20 minutes. After cooling it to -10°C or lower, a solution of 6.14 g (19.1 mmol) of p-mentyl hydroperoxide in 8.5 ml of chlorobenzene was added dropwise thereto at -10°C or lower and the mixture was stirred at -10°C for 17 hours. The reaction mixture was analyzed by HPLC, and the mixture was constituted by 76.4% tert-butyl (2S)-spiro[benzo[c]thiophene-1(3H),4'-piperidine]-1'-carboxylate 2-oxide, 4.4% tert-butyl spiro[benzo[c]thiophene-1(3H),4'-piperidine]-1'-carboxylate 2,2-dioxide and 15.0% tert-butyl spiro[benzo[c]thiophene-1(3H),4'-piperidine]-1'-carboxylate and the enantiomeric excess of the title compound was 69.7%.

### [INDUSTRIAL APPLICABILITY]

The compound of the general formula (1) is an important synthesis intermediate of superior neurokinin receptor antagonists (USP 6159967), and according to the process of the present invention, the compound of the general formula (2) can be obtained more economically and at higher yield than methods of the prior art (USP 6159967 and T. Nishi et al., Tetrahedron Asymmetry, 1998, 9, 2567-2570), and thus the process of the present invention is industrially useful.

## Claims

1. A process for preparing a compound of formula (1): 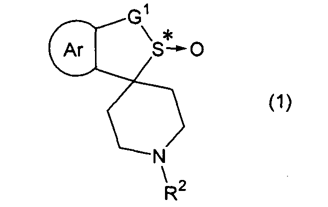 [wherein G¹ represents a C₁-C₆ alkylene group; Ar represents a C₆-C₁₀ aryl group which may be substituted by one or more group(s) selected from Substituent group α or a 5 to 7-membered heteroaryl group containing 1 to 3 sulfur atoms, oxygen atoms and/or nitrogen atoms which may be substituted by one or more group(s) selected from Substituent group α; R² represents a hydrogen atom or an amino protecting group; and Substituent group α consists of a C₁-C₆ alkyl group, a C₁-C₆ alkoxy group and a halogen atom; and * represents an asymmetrical center] or an acid addition salt thereof, which is **characterized by** reacting a compound of formula (2): 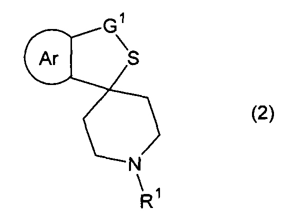 [wherein G¹ and Ar have the same meanings as defined above; R¹ represents an amino protecting group] with cumene hydroperoxide or isopropylcumyl hydroperoxide in the presence of alcohol, water or a mixture of water and alcohol and in the presence of a complex of an optically active tartaric acid diester and a titanium (IV) alkoxide in an inert solvent.

2. The process according to Claim 1, comprising a step of removing R¹, if desired, and carrying out optical resolution, after the oxidation step.

3. The process according to Claim 1, comprising a step of removing R¹, followed by carrying out optical resolution by the diastereomer method, after the oxidation step.

4. The process according to any one of Claims 1 to 3, wherein G¹ is a C₁-C₃ straight alkylene group.

5. The process according to any one of Claims 1 to 3, wherein G¹ is a methylene group.

6. The process according to any one of Claims 1 to 5, wherein Ar is a phenyl group which may be substituted by one or more group(s) selected from Substituent group α.

7. The process according to any one of Claims 1 to 5, wherein Ar is a phenyl group or a phenyl group substituted by 1 or 2 groups selected from the group consisting of fluorine atoms, chlorine atoms, methyl, ethyl, methoxy and ethoxy groups.

8. The process according to any one of Claims 1 to 5, wherein Ar is a phenyl group.

9. The process according to any one of Claims 1 to 8, wherein R¹ is a C₁-C₄ alkanoyl, trifluoroacetyl, methoxyacetyl, benzoyl, 1-naphthoyl, 2-naphthoyl, anisoyl, nitrobenzoyl, C₁-C₄ alkoxycarbonyl, 2,2,2-trichloroethoxycarbonyl, triethylsilylmethoxycarbonyl, 2-(trimethylsilyl)ethoxycarbonyl, vinyloxycarbonyl, allyloxycarbonyl, benzyloxycarbonyl or nitrobenzyloxycarbonyl group.

10. The process according to any one of Claims 1 to 8, wherein R¹ is trifluoroacetyl, methoxycarbonyl, ethoxycarbonyl or tert-butoxycarbonyl.

11. The process according to any one of Claims 1 to 10, wherein R² is a hydrogen atom.

12. The process according to any one of Claims 1 to 11, wherein the titanium (IV) alkoxide is titanium (IV) methoxide, titanium (IV) ethoxide, titanium (IV) propoxide or titanium (IV) isopropoxide.

13. The process according to any one of Claims 1 to 11, wherein the titanium (IV) alkoxide is titanium (IV) isopropoxide.

14. The process according to any one of Claims 1 to 13, wherein the optically active tartaric acid diester is dimethyl (+)- or (-)-tartrate, diethyl (+)- or (-)-tartrate, diisopropyl (+)- or (-)-tartrate, dibutyl (+)- or (-)-tartrate or di-tert-butyl (+)- or (-)-tartrate.

15. The process according to any one of Claims 1 to 13, wherein the optically active tartaric acid diester is diethyl (+)- or (-) -tartrate or diisopropyl (+)- or (-)-tartrate.

16. The process according to any one of Claims 1 to 13, wherein the optically active tartaric acid diester is diisopropyl (+)- or (-)-tartrate.

17. The process according to any one of Claims 2 to 16, wherein the optical resolution agent used in the optical resolution is an optically active sulfonic acid or optically active carboxylic acid.

18. The process according to any one of Claims 2 to 16, wherein the optical resolution agent used in the optical resolution is (+)- or (-)-camphor-10-sulfonic acid, (+)- or (-)-tartaric acid, diacetyl (+)- or (-)-tartaric acid, dibenzoyl (+)- or (-)-tartaric acid, (+)- or (-)-mandelic acid or (+)- or (-)-malic acid.

19. The process according to any one of Claims 1 to 18, wherein the compound of formula (1) is a compound having the S configuration and the optically active tartaric acid diester is dimethyl (-)-tartrate, diethyl (-)-tartrate, diisopropyl (-)-tartrate, dibutyl (-)-tartrate or di-tert-butyl (-)-tartrate and the optical resolution agent used in the case where optical resolution is carried out is (-)-camphor-10-sulfonic acid, (+)-tartaric acid, dibenzoyl (+)-tartaric acid or (+)-mandelic acid.

20. The process according to Claim 19, wherein the optically active tartaric acid diester is diethyl (-)-tartrate or diisopropyl (-)-tartrate.

21. The process according to Claim 19, wherein the optically active tartaric acid diester is diisopropyl (-)-tartrate.

22. A process for preparing a compound of formula (4): 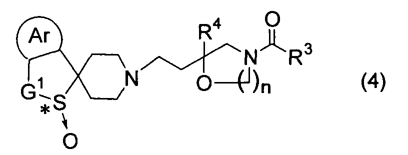 (wherein G¹ represents a C₁-C₆ alkylene group; Ar represents a C₆-C₁₀ aryl group which may be substituted by one or more group(s) selected from Substituent group α or a 5 to 7-membered heteroaryl group containing 1 to 3 sulfur atoms, oxygen atoms and/or nitrogen atoms which may be substituted by one or more group(s) selected from Substituent group α; Substituent group α consists of C₁-C₆ alkyl groups, C₁-C₆ alkoxy groups and halogen atoms; R³ represents a phenyl group substituted by from 1 to 3 groups selected from hydroxyl groups, C₁-C₄ alkoxy groups, halogenated C₁-C₄ alkyl groups and a tetrazolyl group; R⁴ represents a phenyl group substituted by 1 or 2 halogen atoms; n represents 1 or 2; and * represents an asymmetrical center) or a pharmacologically acceptable salt thereof, which substantially consists of the following step A and step B:
{wherein the step A is a step to prepare the compound of formula (1) : 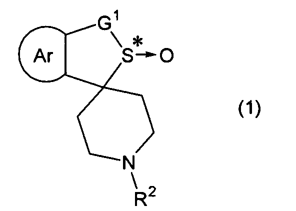 [wherein G¹, Ar and * have the same meanings as defined above; and R² represents a hydrogen atom or a group which is the same group as the group as defined for R¹] or an acid addition salt thereof, by reacting a compound of formula (2): 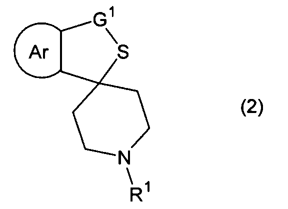 [wherein G¹ and Ar have the same meanings as defined above; and R¹ represents an amino protecting group] with cumene hydroperoxide or isopropylcumyl hydroperoxide in the presence of alcohol, water or a mixture of water and alcohol and in the presence of a complex of an optically active tartaric acid diester and a titanium (IV) alkoxide in an inert solvent; and step B is a step to prepare the compound of formula (4) by removing R² in the case where R² of the compound (1) obtained in step A is an amino protecting group and reacting the compound (1), wherein R² is a hydrogen atom, with the compound of formula (3): 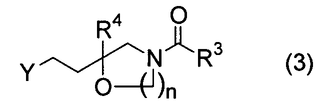 (wherein R³, R⁴ and n have the same meanings as defined above; and Y represents a leaving group).}.

23. The process according to Claim 22, wherein G¹ is a methylene group.

24. The process according to Claim 22 or 23, wherein Ar is a phenyl group.

25. The process according to any one of Claims 22 to 24, wherein R¹ is trifluoroacetyl, methoxycarbonyl, ethoxycarbonyl, tert-butoxycarbonyl or benzyloxycarbonyl.

26. The process according to any one of Claims 22 to 25, wherein R² is a hydrogen atom.

27. The process according to any one of Claims 22 to 26, wherein Y is a halogen atom, a lower alkanesulfonyloxy group, a halogeno lower alkanesulfonyloxy group or an arylsulfonyloxy group.

28. The process according to any one of Claims 22 to 27, wherein n is 2.

29. The process according to any one of Claims 22 to 28, wherein R³ is 3,5-bis(trifluoromethyl)phenyl, 3,4,5-trimethoxyphenyl, 3-hydroxy-4,5-dimethoxyphenyl, 4-hydroxy-3,5-dimethoxyphenyl or 2-methoxy-5-(1-tetrazolyl)phenyl.

30. The process according to any one of Claims 22 to 29, wherein R⁴ is a phenyl group substituted by 1 or 2 fluorine atoms or chlorine atoms.
